# EUROPEAN PATENT SPECIFICATION

(11) **EP 2 265 631 B1**
(45) Date of publication and mention of the grant of the patent: **25.04.2012**
(21) Application number: 09716413.1
(22) Date of filing: 03.03.2009
(51) Int. Cl.: C07K 7/08, A61K 38/10

(54) **NOVEL ANTIMICROBIAL PEPTIDES AND THEIR APPLICATION**
NEUE ANTIMIKROBIELLE PEPTIDE UND IHRE ANWENDUNG
NOUVEAUX PEPTIDES ANTIMICROBIENS ET LEUR APPLICATION

(30) Priority: 04.03.2008 CZ 20080128; 10.06.2008 CZ 20080356
(43) Date of publication of application: 29.12.2010
(73) Proprietor: Ustav Organicke Chemie a Biochemie Akademie Ved Ceske Republiky, V.V.I., 166 10 Praha 6 (CZ)
(72) Inventor: CEROVSKY, Václav, 160 00 Praha 6 (CZ); SLANINOVÁ, Jirina, 147 00 Praha 4 (CZ); HOVORKA, Oldrich, 263 01 Dobrís (CZ); CVACKA, Josef, 110 00 Praha 1 (CZ); VOBURKA, Zdenek, 186 00 Praha 8 (CZ); FUCÍK, Vladimír, 170 00 Praha 7 (CZ); BEDNÁROVÁ, Lucie, 161 00 Praha 6 (CZ); VOTRUBA, Ivan, 182 00 Praha 8 (CZ); BOROVICKOVÁ, Lenka, 130 00 Praha 3 (CZ); TURÁNEK, Jaroslav, 638 00 Brno-Lesná (CZ)
(74) Representative: Herman, Vaclav
(86) International application number: PCT/CZ2009/000034
(87) International publication number: WO 2009/109156

(56) References cited:
- WO-A-95/00547
- WO-A-2007/133153
- SONG YUN MI ET AL: "Cell selectivity and mechanism of action of antimicrobial model peptides containing peptoid residues." BIOCHEMISTRY 13 SEP 2005, vol. 44, no. 36, 13 September 2005 (2005-09-13), pages 12094-12106, XP002562124 ISSN: 0006-2960
- CEROVSKY V ET AL: "New potent antimicrobial peptides from the venom of Polistinae wasps and their analogs" PEPTIDES, ELSEVIER, AMSTERDAM, vol. 29, no. 6, 19 February 2008 (2008-02-19), pages 992-1003, XP022646985 ISSN: 0196-9781 [retrieved on 2008-02-19] cited in the application
- CEROVSKÝ VÁCLAV ET AL: "Melectin: a novel antimicrobial peptide from the venom of the cleptoparasitic bee Melecta albifrons." CHEMBIOCHEM : A EUROPEAN JOURNAL OF CHEMICAL BIOLOGY 24 NOV 2008, vol. 9, no. 17, 24 November 2008 (2008-11-24), pages 2815-2821, XP002562125 ISSN: 1439-7633 cited in the application

## Description

### Technical field

The invention involves a novel peptide, isolated primarily from natural source, of the formula I
H-Gly-Phe-Leu-Ser-Ile-Leu-Lys-Lys-Val-Leu-Pro-Lys-Val-Met-Ala-His-Met-Lys-NH₂ (I),
its new synthetic analogs,
and their applications as antimicrobial, antiviral, antifungal, antiparasitic and anticancer compounds.

### Background arts

Antimicrobial peptides are evolutionary conserved components of the host's innate immunity system that form the first line of defense against infections [1-3]. They have been identified in almost all classes of life. Among antimicrobial peptides, those from insects constitute a remarkable group [4]. Since insects are uniquely adapted to a variety of natural environments, often considered rather unhealthy by human standards, they have developed amazing resistance to bacterial infection, in which antimicrobial peptides play a major role. The significant advantage of antimicrobial peptides resides in their mechanism of action, which is markedly different from that of conventional antibiotics. Although the precise mechanism of the broad spectrum of antimicrobial activity of these peptides is not yet fully understood, they appear to act via a specific, but not receptor-mediated, formation of transmembrane pores or ion channels on cellular membrane. This causes leakage of essential metabolites that results in the disruption of microbial cell structure and leads to cell death [5, 6]. In contrast to conventional antibiotics, they do not appear to induce microbial resistance and require only short time to induce killing. Quite peculiar groups of peptides with antimicrobial properties were identified in the venom of stinging insects such as hymenopterans [7]. The main function of hymenoptera venom is the protection against predators or nest defense against intruders causing them pain and inflammation. Antimicrobial effect of peptides isolated from the venoms of wasps, bees, bumblebees and ants is rather secondary as in the case of peptides belonging to the group of mastoparans [8]. Nevertheless, antimicrobial effect of peptides isolated for example from the venom of wild bees is quite significant. As the growing resistance of bacterial pathogens to conventional antibiotics has become serious global health problem, this alarming situation resulted in a search for novel alternative to traditional antibiotics such as antimicrobial peptides. As regards of the synthetic availability of peptides isolated from the venom of wild bees, these peptides may found application in practical medicine.

### Disclosure of the invention

The invention involves a novel peptide isolated primarily from natural source of the formula I
H-Gly-Phe-Leu-Ser-Ile-Leu-Lys-Lys-Val-Leu-Pro-Lys-Val-Met-Ala-His-Met-Lys-NH₂ (I),
and its new synthetic analogs of formulas II/1 to II/18, obtainable by a replacement of several amino acid residues, by an insertion or omitting of them or by an alteration of the amino acid residues order.

This invention further involves the listed peptide of formula I and its synthetic analogs of formulas II/1 to II/18 for use as antimicrobial, antiviral, antifungal, antiparasitic and anticancer compounds.

### Detailed description of the invention

We isolated the peptide of the formula (I) from the venom reservoirs of cleptoparasitic bees *Melecta albifrons* and named it melectin. The peptides of formulas (II/1 to II/18) are analogs of peptide (I). The peptide I possess strong antimicrobial effect and low hemolytic activity, but its primary function in the nature is unknown.

Even if this primary role of melectin in the nature is not known, we deal with novel unique, so far not published, peptide with significant antimicrobial properties. This peptide shows antimicrobial properties thanks to the shape of its secondary structure, which depends on its distinctive primary peptide sequence. Secondary structure of melectin is unordered in aqueous media, but it easily undergoes to the α-helical structure in the presence of α-helix inducing compounds such as trifluoroethanol or SDS or in an anisotropic environment of the bacterial cell membrane [9].

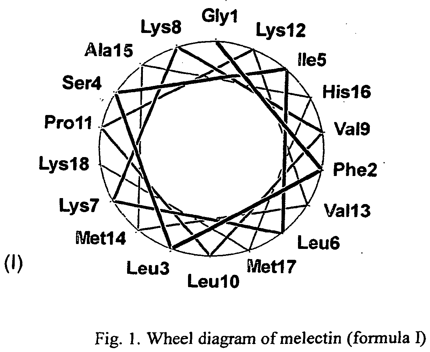

In the so called Edmundson wheel [10] projection (Fig. 1), melectin [9] have well-defined hydrophobic sectors with large aliphatic residues (in gray frames) on one side of the helix, and hydrophilic sector (residues without frames) dominated by cationic Lys residues on the opposite side of the helix. The ability of this peptide to adopt such an amphipathic structure [11, 12] within bacterial cell membrane is one of the most important factors participating in the disruption of bacterial cell membrane finally resulting in the bacteria death. In addition, the cationic character [11] of this peptide ensured by the presence of four lysine residues in melectin [9] located in hydrophilic sectors of the helices, plays also important role in the killing process. Due to this space arrangement, the positively charged faces of the peptide are attracted by strong electrostatic interaction to negatively charged phospholipids of bacterial cell membrane. The peptide accumulation on the membrane surface causes tension between the two leaflets of the lipidic membrane bilayer, which above a threshold peptide concentration leads to the disintegration and rupture of the membrane.

In anisotropic environment melectin may theoretically form the optimal α-helical structures of five and four turns respectively. These helices are stabilized by hydrogen bonds in which every backbone NH group donates a hydrogen bond to the backbone C=O group of the preceding turn. In the case of melectin, due to the presence of proline residue (Pro) in position 11, the possibility of making hydrogen bond to the preceding turn is lost since proline lacks the hydrogen on its amide and because of a steric effect of its ring structure. As a result a kink [9, 13, 14] is introduced to the helical structure [9] causing slight bending of α-helix. This conformational element, imposed by proline on a melectin peptide chain, appears to govern its biological functions such as high antimicrobial activity and low hemolytic effect. The replacement of Pro by other amino acid residue in the synthetic peptides results in the increase of undesirable hemolytic activity (Table 1). Such effect has been already described in the case of other antimicrobial peptides [13, 14]. The entire cationicity, hydrophobicity or amphipathicity of melectin can be modified by the replacement of the original amino acid residues in its sequence by other amino acids. This is done in an effort to increase its antimicrobial activity and reduce hemolytic activity [15]. Other different chemical modifications as demonstrated by general formulas II/1 - II/18 influence the biological properties of melectin. Tables 1 - 4 show the examples of chemical modifications of peptides and their consequences on antimicrobial and hemolytic activity.

### Examples

### Example 1

### Isolation from natural sources

Bee specimens of *Melecta albifrons* were collected in urban area of north-west quarter of Prague, Czech Republic, and kept frozen at -20°C for several days. The venom reservoirs of four individuals were removed by dissection and their contents was extracted with 25 µl of a mixture of acetonitrile-water (1:1) containing 0.1% TFA. The extract was centrifuged, and the supernatant was fractionated by RP-HPLC. The chromatography was carried out on the Thermo Separation Product instrument with a Vydac C-18 column (250 x 4.6 mm; 5 µm) at a flow rate 1.0 ml/min using the gradient of solvents from 5% to 70% acetonitrile/water/0.1% TFA over 60 min. The major peptide fractions detected by the UV absorption at 222 nm were collected, the solvent was evaporated in a Speed-vac and the material was subjected to mass spectrometry and Edman degradation to determine its primary structure. Melectin represented the main peptide component of the venom extract.

### Example 2

### Synthetic preparation of peptides of the formulas I and II/1 to II/18

These peptides may be synthesized according to following procedure: peptide was synthesized manually by using a solid-phase method in 5 mL polypropylene syringes with a bottom Teflon filter. Synthesis was done by using a Fmoc chemistry protocol on a Rink Amide MBHA resin (100 mg) with 0.7 mmol/g substitution. Protected amino acids were coupled in fourfold excess in dimethylformamide as solvent and N,N'-diisopropylcarbodiimide (7 equivalents)/1-hydroxybenzotriazole (5 equivalents) as coupling reagents. Crude peptides of the formulas I and II/1 to II/18 were deprotected and cleaved from the resin with a mixture of trifluoroacetic acid/1,2-ethanedithiol/water/thioanisol/triisopropylsilane (90 : 2.5 : 2.5 : 3 : 2) for 3.5 h and precipitated with *tert-*butyl methyl ether. Usually 100 - 150 mg of crude peptides was obtained by this manner. Crude peptides (15 mg) were purified by preparative RP-HPLC using a Vydac C-18 column (250 x 10 mm) at a flow rate 3.0 ml/min using the gradient of solvents from 5% to 70% acetonitrile/water/0.1% TFA over 60 min yielding 7 - 9 mg of HPLC pure peptides. Their identities were verified by mass spectrometry as given in Table 1.

**Table 1. Amino acid sequences and MS data of melectin (I) and its synthetic analogs of formulas II/1 to II/18**

| | | | |
|---|---|---|---|
| Peptide | Peptide sequence | Monoisotopic molecular mass (Da) | |

| | | calculated | found |
|---|---|---|---|
| (I) | H-Gly-Phe-Leu-Ser-Ile-Leu-Lys-Lys-Vai-Leu-Pro-Lys-Val-Met-Ala-His-Met-Lys-NH₂ | 2038.23 | 2038.2 |
| II/1 | H-Gly-Phe-Leu-Ser-Ile-Leu-Lys-Lys-Val-Leu-**Lys**-Lys-Val-Met-Ala-His-Met-Lys-NH₂ | 2069.27 | 2069.4 |
| II/2 | H-Gly-Phe-Leu-Ser-Ile-Leu-Lys-Lys-Val-Leu-**Ala**-Lys-Val-Met-Ala-His-Met-Lys-NH₂ | 2012.22 | 2012.3 |
| II/3 | H-Gly-Phe-Leu-Ser-lle-Leu-Lys-Lys-Val-Leu-**Gly**-Lys-Val-Met-Ala-His-Met-Lys-NH₂ | 1998.20 | 1998.2 |
| II/4 | H-Gly-Phe-Leu-Ser-Ile-Leu-Lys-Lys-Val-Leu-Pro-Lys-Val-Met-Ala-Lys-Met-Lys-NH₂ | 2029.27 | 2029.4 |
| II/5 | H-Gly-Phe-Leu-Ser-Ile-Leu-Lys-Lys-Val-Leu-Pro-Lys-Val-**Leu**-Ala-His-**Leu**-Lys-NH₂ | 2002.32 | 2002.4 |
| II/6 | H-Gly-Phe-Leu-Ser-Ile-Leu-Lys-Lys-Val-Leu-Pro-Lys-Val-Met-His-Ala-Met-Lys-NH₂ | 2038.23 | 2038.3 |
| II/7 | H-Gly-Phe-Leu-Ser-Ile-Leu-Lys-Lys-Val-Leu-Pro-Lys-Val-Met-Lys-Ala-Met-Lys-NH₂ | 2029.27 | 2029.4 |
| II/8 | H-Gly-Phe-Leu-Ser-Ile-Leu-Lys-Lys-Val-Leu-... -Lys-Val-Met-Ala-His-Met-Lys-NH₂ | 1941.18 | 1940.9 |
| II/9 | H-Phe-Leu-Ser-Ile-Leu-Lys-Lys-Val-Leu-Pro-Lys-Val-Met-Ala-His-Met-Lys-NH₂ | 1981.21 | 1980.7 |
| II/10 | H-Leu-Ser-Ile-Leu-Lys-Lys- Val-Leu-Pro-Lys-Val-Met-Ala-His-Met-Lys-NH₂ | 1834.14 | 1833.8 |
| II/11 | H-Ser-Ile-Leu-Lys-Lys-Val-Leu-Pro-Lys-Val-Met-Ala-His-Met-Lys-NH₂ | 1721.06 | 1720.7 |
| II/12 | H-Gly-Phe-Leu-Ser-Ile-Leu-Lys-Lys-Val-Leu-Pro-NH₂ | 1212.80 | 1212.7 |
| II/13 | H-Gly-Phe-Leu-Ser-Ile-Leu-Lys-Lys-Val-Leu-NH₂ | 1115.74 | 1115.6 |
| II/14 | H-Gly-Phe-Leu-Ser-Ile-Leu-Lys-Lys-Val-**Gly**-Pro-Lys-Val-Met-Ala-His-Met-Lys-NH₂ | 1982.17 | 1982.0 |
| II/15 | H-Gly-Phe-Leu-Ser-Ile-Leu-Lys-Lys-Val-Leu-***Pro***-Lys-Val-Met-Ala-His-Met-Lys-NH₂ | 2038.23 | 2038.0 |
| II/16 | H-Gly-**1Nal**-Leu-Ser-Ile-Leu-Lys-Lys- V al-Leu-Pro-Lys- V al-Met-Ala-His-Met-Lys-NH₂ | 2088.25 | 2088.0 |
| II/17 | H-Gly-...-Leu-Ser-Ile-Leu-Lys-Lys-Val-Leu-**Lys**-Lys-Val-Met-Ala-His-Met-Lys-NH₂ | 1923.15 | 1923.6 |
| II/18 | H-Gly-Phe-Leu-Ser-Ile-Leu-Lys-Lys-Val-Leu-Pro-Lys-Val-**Nle**-Ala-His-**Nle**-Lys-NH₂ | 2002.32 | 2002.5 |

| | | | |
|---|---|---|---|
| ***Pro**:* D-prolin; **1Nal**: 3-(1-naftyl)alanin; **Nle**: norleucine; bold letters: amino acid residue replacement; underlined: interchanged amino acid residues; -...- the residue was omitted | | | |

### Example 3

### Determination of antimicrobial activity

Antimicrobial activity was determined as a quantitative minimum inhibitory concentration (MIC) by observing bacterial growth in the presence of different concentrations of tested peptides. Mid-exponential phase bacteria were added to the solutions of the tested antimicrobial peptide at different concentrations (0.5 to 100 µM) in LB broth in multi-well plates. The plates were incubated at 37°C for 20 h while being continuously shaken in a Bioscreen C instrument (Finland). The absorbance was measured at 540 nm every 15 min and each peptide was tested at least 3 times in duplicate. 1.2.10³ - 7.5.10³ CFU of bacteria was routinely used in the experiments. Tetracycline in a concentration range of 0.1 - 50 µM was tested as a standard. The results are shown in Table 3.

### Example 4

### Determination of hemolytic activity

The peptides in the concentration (1-200 µM) were incubated with rat red blood cells (5%) for 1 h at 37°C in a physiological solution at a final volume of 0.2 ml. The samples were then centrifuged for 5 min at 250 g, and the absorbance of the supernatant was determined at 540 nm. Supernatants of red blood cells suspended in physiological solution and in 0.2% TRITON X100 in physiological solution served as controls for zero hemolysis (blank) and 100% hemolysis, respectively. The results are shown in Table 3.

**Table 3. Antimicrobial and hemolytic activity of melectin (I) and its synthetic analogs of formulas II/1 to II/18**

| Peptide MIC [µM] | Antimicrobial activity IC₅₀ [µM] | | | | Hemolytic activity |
|---|---|---|---|---|---|
| | *B.s.* | *S.a.* | *E. c.* | *P.a.* | |
| I | 0.8 | 6.8 | 2.0 | 18.5 | > 100 |
| II/1 | 1.4 | 13.3 | 1.8 | 25.3 | 27.3 |
| II/2 | 1.3 | 4.7 | 1.6 | 18.3 | 29.7 |
| II/3 | 1.3 | 4.3 | 1.8 | 14.4 | 41.4 |
| II/4 | 0.7 | 16.8 | 2.1 | 33.6 | >100 |
| II/5 | 0.8 | 5.0 | 1.2 | 20 | 43.7 |
| II/6 | 1.1 | 9.1 | 2.1 | 35.1 | 150 |
| II/7 | 0.8 | 7.7 | 1.5 | 31.7 | 70 |
| II/8 | 0.9 | 5.0 | 1.8 | 27.5 | 22.9 |
| II/9 | 1.2 | 38.8 | 9.08 | 62.1 | >200 |
| II/10 | 7.9 | > 100 | 76.2 | > 100 | »200 |
| II/11 | 21.8 | > 100 | > 100 | > 100 | »200 |
| II/12 | 13.0 | > 100 | 78.0 | > 100 | »200 |
| II/13 | 2.9 | 10.9 | 9.1 | 58.7 | >100 |
| II/14 | 1.4 | 80.0 | 22.5 | 80.0 | »200 |
| II/15 | 1.7 | >100 | 4.2 | 100 | »200 |
| II/16 | 0.4 | 2.5 | 0.4 | 17.3 | 25 |
| II/17 | 0.95 | 40 | 3.8 | 10 | »200 |
| II/18 | 0.2 | 2.5 | 0.75 | 17.3 | 50 |

| | | | | | |
|---|---|---|---|---|---|
| *B.s,. Bacilus subtilis; S.a., Staphylococcus aureus; E.c., Escherichia coli; P.a., Pseudomonnas aeruginosa* | | | | | |

### Example 5

### Cytostatic effect on cancer cells

Tumor cells in suspension or adherent cells were incubated under standard conditions in appropriate culture media for 24-72 h with the peptides in 2-40 micromolar concentration. After this time, the number of living cells was determined using routine tests, e.g. MTT test. The IC₅₀ was determined as concentration of peptides, which caused 50% decrease in cell viability in comparison to controls (no peptide present). In preliminary tests mouse lymphocytic leukemia L1210 (CCL 219), CCRF-CEM T lymphoblastoid (human acute lymphoblastic leukemia, CCL 119), human promyelocytic leukemia HL-60 (CCL 240), human cervix carcinoma HeLa S3, rat pheochromocytoma PC12, human colon adenocarcinoma SW480 (CCL-228), and normal rat epithelial cells (IEC-6, CRL-1592) from ATCC (Manassas, VA, USA), were used. Peptides melectin and one of its analogs showed IC₅₀ in the range of 4-30 mM (Table 4).

**Table 4. Cytostatic effect of selected peptides on cancer cells**

| Peptide | IC₅₀ [µM] | | | | | |
|---|---|---|---|---|---|---|
| | L1210 | CCRF-CEM T | HL-60 | HeLaS3 | PC12 | SW480 |
| I | >10 | >10 | >10 | 18.1 | 10 | 25 |
| II/7 | 6.9 | 8.8 | 9.1 | 9.6 | - | 12.5 |

### References

1. Zasloff M. Antimicrobial peptides of multicellular organisms. Nature 415, 389-395 (2002)
2. Hancock R.E.W., Sahl H.-G. Antimicrobial and host-defense peptides as new anti-infective therapeutic strategies. Natur. Bitech. 24, 1551-1557 (2006)
3. Toke O. Antimicrobial peptides: New candidates in the fight against bacterial infection. Biopolymers (Peptide Science) 80, 717-735 (2005).
4. Otvos L. Jr. Antimicrobial peptides isolated from insects. J. Peptide Sci. 6, 497-511 (2000)
5. Oren Z, Shai Y. Mode of action of linear amphipathic α-helical antimicrobial peptides. Biopolymers 47, 451-463 (1998)
6. Yeaman M.R., Yount N.Y. Mechanisms of antimicrobial peptide action and resistance. Pharm. Rev. 55, 27-55 (2003)
7. Kuhn-Nentwig L. Antimicrobial and cytolytic peptides of venomous arthropods. Cell. Mol. Life Sci. 60, 2651-2668 (2003)
8. 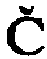 e 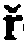 ovsk 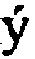 V., Slaninová J., Fu 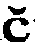 ík V, Hula 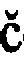 ová H, Borovi 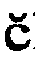 ková L., Je 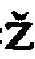 ek R and Bednárová L. New potent antimicrobial peptides from the venom of Polistinae wasps and their analogs. Peptides 29, 992-1003 (2008)
9. 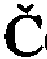 e 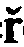 ovsk 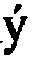 V., Hovorka O., Cva 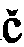 ka J., Voburka Z., Bednárová L., Borovi 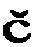 ková L., Slaninová J., and Fu 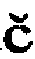 ík V. Melectin: a novel antimicrobial peptide from the venom of the cleptoparasitic bee Melecta albifrons. ChemBioChem 9, 2815-2821 (2008)
10. Schiffer M. and Edmundson A. B. Use of helical wheels to represent the structures of proteins and to identify segments with helical potential. Biophys. J. 7, 121-135 (1967)
11. Tossi A., Sandri L., Giangaspero A. Amphipathic, α-helical antimicrobial peptides. Biopolymers (Peptide Science) 55, 4-30 (2000)
12. Pathak N., Salas-Auvert R., Ruche G., Janna M.-H., McCarthy D., Harrison R.G. Comparison of the effect of hydrophobicity, amphiphilicity, and α-helicity on the activities of antimicrobial peptides. Proteins: Struct. Funct. Genet. 22; 182-86 (1995)
13. Suh J.-Y., Lee Y.-T., Park C.-B., Lee K.-H., Kim S.-C., Choi B.-S. Structural and functional implications of a proline residue in the antimicrobial petide gaegurin. Eur. J. Biochem. 266, 665-674 (1999)
14. Shin S.Y., Lee S.-H., Yang S.-T., Park E.J., Lee D.G., Lee M.K., Eom S.H., Song W.K., Kim Y., Hahm K.-S., Kim J.Y. Antibacterial, antitumor and hemolytic activities of α-helical antibiotik peptide, P18 and its analogs. J. Pept. Res.58, 504-514 (2001)
15. Conlon J.M., Al-Ghaferi N., Abraham B. and Leprince J. Strategies for transformation of naturally-occurring amphibian antimicrobial peptides into therapeutically valuable anti-infective agents. Methods 42, 349-357 (2007)

## Claims

1. Peptid of the formula I
H-Gly-Phe-Leu-Ser-Ile-Leu-Lys-Lys-Val-Leu-Pro-Lys-Val-Met-Ala-His-Met-Lys-NH₂ (I).

2. Synthetic analogs of peptide I described in claim 1, having the formulas II/1 to II/18
H-Gly-Phe-Leu-Ser-Ile-Leu-Lys-Lys-Val-Leu-Lys-Lys-Val-Met-Ala-His-Met-Lys-NH₂ (II/1),
H-Gly-Phe-Leu-Ser-Ile-Leu-Lys-Lys-Val-Leu-Ala-Lys-Val-Met-Ala-His-Met-Lys-NH2 (II/2),
H-Gly-Phe-Leu-Ser-Ile-Leu-Lys-Lys-Val-Leu-Gly-Lys-Val-Met-Ala-His-Met-Lys-NH₂ (II/3),
H-Gly-Phe-Leu-Ser-Ile-Leu-Lys-Lys-Val-Leu-Pro-Lys-Val-Met-Ala-Lys-Met-Lys-NH₂ (II/4),
H-Gly-Phe-Leu-Ser-Ile-Leu-Lys-Lys-Val-Leu-Pro-Lys-Val-Leu-Ala-His-Leu-Lys-NH₂ (II/5),
H-Gly-Phe-Leu-Ser-Ile-Leu-Lys-Lys-Val-Leu-Pro-Lys-Val-Met-His-Ala-Met-Lys-NH₂ (II/6),
H-Gly-Phe-Leu-Ser-Ile-Leu-Lys-Lys-Val-Leu-Pro-Lys-Val-Met-Lys-Ala-Met-Lys-NH₂ (II/7),
H-Gly-Phe-Leu-Ser-Ile-Leu-Lys-Lys-Val-Leu-Lys-Val-Met-Ala-His-Met-Lys-NH2 (II/8),
H-Phe-Leu-Ser-Ile-Leu-Lys-Lys-Val-Leu-Pro-Lys-Val-Met-Ala-His-Met-Lys-NH₂ (II/9),
H-Leu-Ser-Ile-Leu-Lys-Lys-Val-Leu-Pro-Lys-Val-Met-Ala-His-Met-Lys-NH₂ (II/10),
H-Ser-Ile-Leu-Lys-Lys-Val-Leu-Pro-Lys-Val-Met-Ala-His-Met-Lys-NH₂ (II/11),
H-Gly-Phe-Leu-Ser-Ile-Leu-Lys-Lys-Val-Leu-Pro-NH₂ (II/12),
H-Gly-Phe-Leu-Ser-Ile-Leu-Lys-Lys-Val-Leu-NH₂ (II/13),
H-Gly-Phe-Leu-Ser-Ile-Leu-Lys-Lys-Val-Gly-Pro-Lys-Val-Met-Ala-His-Met-Lys-NH₂ (II/14),
H-Gly-Phe-Leu-Ser-Ile-Leu-Lys-Lys-Val-Leu-D-Pro-Lys-Val-Met-Ala-His-Met-Lys-NH₂ (II/15),
H-Gly-lNal-Leu-Ser-Ile-Leu-Lys-Lys-Val-Lu-Pro-Lys-Val-Met-Ala-His-Met-Lys-NH₂ (II/16),
H-Gly-Leu-Ser-Ile-Leu-Lys-Lys-V al-Leu-Lys-Lys-V al-Met-Ala-His-Met-Lys-NH₂ (II/17),
H-Gly-Phe-Leu-Ser-Ile-Leu-Lys-Lys-Val-Leu-Pro-Lys-Val-Nle-Ala-His-Nle-Lys-NH₂ (II/18).

3. Peptides of the formulas I and II/1 to II/18 described in the claims 1 and 2 for use as antimicrobial, antiviral, antifungal, antiparasitic and anticancer compounds.

4. Peptides according to claims 1 or 2 for use in the production of the medication for the treatment of microbial, viral, parasitic and fungal diseases and for the treatment of cancer diseases.

## Patentansprüche

1. Peptid der Formel I
H-Gly-Phe-Leu-Ser-Ile-Leu-Lys-Lys-Val-Leu-Pro-Lys-Val-Val-Met-Ala-His-Met-Lys-NH₂ (I).

2. Die synthetischen Analogen des in Anspruch 1 definierten Peptids I haben die Formeln II/1 bis II/18
H-Gly-Phe-Leu-Ser-Ile-Leu-Lys-Lys-Val-Leu-Lys-Lys-Val-Met-Ala-His-Met-Lys-NH₂ (II/1).
H-Gly-Phe-Leu-Ser-Ile-Leu-Lys-Lys-Val-Leu-Ala-Lys-Val-Met-Ala-His-Met-Lys-NH₂ (II/2),
H-Gly-Phe-Leu-Ser-Ile-Leu-Lys-Lys-Val-Leu-Gly-Lys-Val-Met-Ala-His-Met-Lys-NH₂ (II/3).
H-Gly-Phe-Leu-Ser-Ile-Leu-Lys-Lys-Val-Leu-Pro-Lys-Val-Met-Ala-Lys-Met-Lys-NH₂ (II/4),
H-Gly-Phe-Leu-Ser-Ile-Leu-Lys-Lys-Val-Leu-Pro-Lys-Val-Leu-Ala-His-Leu-Lys-NH₂ (II/5).
H-Gly-Phe-Leu-Ser-Ile-Leu-Lys-Lys-Val-Leu-Pro-Lys-Val-Met-His-Ala-Met-Lys-NH₂ (II/6),
H-Gly-Phe-Leu-Ser-Ile-Leu-Lys-Lys-Val-Leu-Pro-Lys-Val-Met-Lys-Ala-Met-Lys-NH₂ (II/7),
H-Gly-Phe-Leu-Ser-Ile-Leu-Lys-Lys-Val-Leu-Lys-Val-Met-Ala-His-Met-Lys-NH₂ (II/8),
H-Phe-Leu-Ser-lIe-Leu-Lys-Lys-Val-Leu-Pro-Lys-Val-Met-Ala-His-Met-Lys-NH₂ (II/9),
H-Leu-Ser-Ile-Leu-Lys-Lys-Val-Leu-Pro-Lys-Val-Met-Ala-His-Met-Lys-NH₂ (II/10),
H-Ser-Ile-Leu-Lys-Lys-Val-Leu-Pro-Lys-Val-Met-Ala-His-Met-Lys-NH₂ (II/11),
H-Gly-Phe-Leu-Ser-Ile-Leu-Lys-Lys-Val-Leu-Pro-NH₂ (II/12),
H-Gly-Phe-Leu-Ser-Ile-Leu-Lys-Lys-Val-Leu-NH₂ (II/13),
H-Gly-Phe-Leu-Ser-Ile-Leu-Lys-Lys-Val-Gly-Pro-Lys-Val-Met-Ala-His-Met-Lys-NH₂ (II/14);
H-Gly-Phe-Leu-Ser-Ile-Leu-Lys-Lys-Val-Leu-D-Pro-Lys-Val-Met-Ala-His-Met-Lys-NH₂ (II/15),
H-Gly-1Nal-Leu-Ser-Ile-Leu-Lys-Lys-Val-Leu-Pro-Lys-Val-Met-Ala-His-Met-Lys-NH₂ (II/16).
H-Gly-Leu-Ser-Ile-Leu-Lys-Lys-Val-Leu-Lys-Lys-Val-Met-Ala-His-Met-Lys-NH₂ (II/17),
H-Gly-Phe-Leu-Ser-Ile-Leu-Lys-Lys-Val-Leu-Pro-Lys-Val-Nle-Ala-His-Nle-Lys-NH₂ (II/18).

3. Peptide der in Ansprüchen 1 und 2 beschriebenen Formeln I und II/1 bis II/18 zur Anwendung als antimikrobielle; antivirale; fungizide, antiparasitische und Antikrebsverbindungen.

4. Peptide nach Ansprüchen 1 oder 2 zur Anwendung bei der Herstellung von Arzneimitteln zur Behandlung vor mikrobiellen, parasitischen sowie Virus-, Pilz- und Krebserkrankungen.

## Revendications

1. Peptide de la formule I
H-Gly-Phe-Leu-Ser-Ile-Leu-Lys-Lys-Val-Leu-Pro-Lys-Val-Met-Ala-His-Met-Lys-NH₂ (I).

2. Analogue synthétiques de peptide 1 qui est décrit dans la revendication 1, ayant les formules de II/1 à II/18
H-Gly-Phe-Leu-Ser-Ile-Leu-Lys-Lys-Val-Leu-Lys-Lys-Val-Met-Ala-His-Met-Lys-NH₂ (II/1),
H-Gly-Phe-Leu-Ser-Ile-Leu-Lys-Lys-Val-Leu-Ala-Lys-Val-Met-Ala-His₋Met-Lys-NH₂ (II/2),
H-Gly-Phe-Leu-Ser-Ile-Leu-Lys-Lys-Val-Leu-Gly-Lys-Val-Met-Ala-His-Met-Lys-NH₂ (II/3),
H-Gly-Phe-Leu-Ser-Ile-Leu-Lys-Lys-Val-Leu-Pro-Lys-Val-Met-Ala-Lys-Met-Lys-NH₂ (II/4),
H-Gly-Phe-Leu-Ser-Ile-Leu-Lys-Lys-Val-Leu-Pro-Lys-Val-Leu-Ala-His-Leu-Lys-NH₂ (II/5),
H-Gly-Phe-Leu-Ser-Ile-Leu-Lys-Lys-Val-Leu-Pro-Lys-Val-Met-His-Ala-Met-Lys-NH₂ (II/6),
H-Gly-Phe-Leu-Ser-Ile-Leu-Lys-Lys-Val-Leu-Pro-Lys-Val-Met-Lys-Ala-Met-Lys-NH₂ (II/7),
H-Gly-Phe-Leu-Ser-Ile-Leu-Lys-Lys-Val-Leu-Lys-Val-Met-Ala-His-Met-Lys-NH₂ (II/8),
H-Phe-Leu-Ser-Ile-Leu-Lys-Lys-Val-Leu-Pro-Lys-Val-Met-Ala-His-Met-Lys-NH₂ (II/9),
H-Leu-Ser-Ile-Leu-Lys-Lys-Val-Leu-Pro-Lys-Val-Met-Ala-His-Met-Lys-NH₂ (II/10),
H-Ser-Ile-Leu-Lys-Lys-Val-Leu-Pro-Lys-Val-Met-Ala-His-Met-Lys-NH₂ (II/11),
H-Gly-Phe-Leu-Ser-Ile-Leu-Lys-Lys-Val-Leu-Pro-NH₂ (II/12),
H-Gly-Phe-Leu-Ser-Ile-Leu-Lys-Lys-Val-Leu-NH₂ (II/13),
H-Gly-Phe-Leu-Ser-Ile-Leu-Lys-Lys-Val-Gly-Pro-Lys-Val-Met-Ala-His-Met-Lys-NH₂ (II/14),
H-Gly-Phe-Leu-Ser-Ile-Leu-Lys-Lys-Val-Leu-D-Pro-Lys-Val-Met-Ala-His-Met-Lys-NH₂ (II/15),
H-Gly-lNal-Leu-Ser-Ile-Leu-Lys-Lys-Val-Leu-Pro-Lys-Val-Met-Ala-His-Met-Lys-NH₂ (II/16),
H-Gly-Leu-Ser-Ile-Leu-Lys-Lys-Val-Leu-Lys-Lys-Val-Met-Ala-His-Met-Lys-NH₂ (II/17),
H-Gly-Phe-Leu-Ser-Ile-Leu-Lys-Lys-Val-Leu-Pro-Lys-Val-Nle-Ala-His-Nle-Lys-NH₂ (II/18).

3. Peptides des formules I et II/1 à II/18 décrites dans les revendications 1 et 2 pour l'usage en tant que composés antimicrobiens, antiviraux, antifongiques, antiparasitaires et anticancéreux.

4. Peptides selon les revendications 1 ou 2 pour l'usage dans la production du médicament pour le traitement des maladies microbiennes, virales, parasitiques et fongiques et pour le traitement des maladies de cancer.
